# EUROPEAN PATENT APPLICATION

(11) **EP 2 172 201 A1**
(43) Date of publication of application: **07.04.2010**
(21) Application number: 07851069.0
(22) Date of filing: 21.12.2007
(51) Int. Cl.: A61K 31/4725, A61K 31/18, A61P 13/02, A61P 13/08

(54) **PHARMACEUTICAL COMPOSITION FOR AMELIORATION OF LOWER URINARY TRACT SYMPTOM ASSOCIATED WITH PROSTATOMEGALY**

(30) Priority: 20.07.2007 US 929996 P
(71) Applicant: Astellas Pharma Inc., Tokyo 103-8411 (JP)
(72) Inventor: KAKIZAKI, Hidehiro, Asahikawa-shi Hokkaido 078-8510 (JP); YOSHIDA, Masaki, Kumamoto-shi Kumamoto 860-8556 (JP); UCHIDA; Takeshi, Tokyo 1038411 (JP)
(74) Representative: HOFFMANN EITLE
(86) International application number: PCT/JP2007/074717
(87) International publication number: WO 2009/013846

(57) **Abstract**

The present invention provides a pharmaceutical composition whose active ingredients are (R)-5-(2-{[2-(2-ethoxyphenoxy)ethyl]amino}propyl-2-methoxybenzene-1-sulfonamide (tamsulosin), or its pharmaceutically acceptable salt, and (1S)-1-phenyl-1,2,3,4-tetrahydroisoquanoline-2-carboxylic acid (3R)-quinuclidin-3-yl ester (solifenacin), or its pharmaceutically acceptable salt, particularly a pharmaceutical composition for improvement of lower urinary tract symptoms associated with prostatic hypertrophy. It also provides combination use and combination therapy to improve lower urinary tract symptoms associated with prostatic hypertrophy, using tamsulosin, or its pharmaceutically acceptable salt, and solifenacin, or its pharmaceutically acceptable salt.

## Description

### Technical Field

The present invention relates to a drug, particularly a pharmaceutical composition useful for improvement of lower urinary tract symptoms associated with prostatic hypertrophy, particularly a pharmaceutical composition containing, as active ingredients, (R)-5-(2-{[2-(2-ethoxyphenoxy)ethyl]amino}propyl-2-methoxybenzene-1-sulfonamide (hereinafter referred to as tamsulosin) or its pharmaceutically acceptable salt, and (1S)-1-phenyl-1,2,3,4-tetrahydroisoquinoline-2-carboxylic acid (3R)-quinuclidin-3-yl ester (hereinafter referred to as solifenacin) or its pharmaceutically acceptable salt. The present invention also relates to combination use of tamsulosin or its pharmaceutically acceptable salt and solifenacin or its pharmaceutically acceptable salt, and combination therapy for improvement of lower urinary tract symptoms associated with prostatic hypertrophy.

### Background Art

The prostate gland is a male organ surrounding the urethra between the neck of the bladder and the external urethral sphincter muscle. Prostatic adenoma observed in prostatic hypertrophy is a benign tumor originating from the transitional zone (internal gland) of the prostate, and its growth depends on male hormones (androgens), but its detailed etiological mechanisms have yet to be clarified.
Development of prostatic hypertrophy is considered to arise mainly from direct urethral compression (mechanical occlusion) due to hypertrophy of the adenoma and from an intra-urethral pressure increase (functional occlusion) due to excessive contraction of the prostate and urethra via sympathetic nerves, and the like. The incidence af histologically defined prostatic hypertrophy increases with age, and has been reported to be observed in 90% of 70-80 year-old men.
Dysuria associated with prostatic hypertrophy is considered to arise from the above--mentioned hypertrophy of the prostate and also from excessive contraction of the prostate and urethra, and appears as a wide variety of lower urinary tract symptoms such as voiding symptoms (weakened stream, interrupted stream, delayed urination, abdominal pressure urination, and the like.), storage symptoms (pollakiuria, nocturia, urgency, and the like.), and post-voiding symptoms (emptying, dribbling after urination, and the like.). Therefore, the severity of prostatic hypertrophy is judged by various criteria such as urinary flow-metric tests, prostate weight, and the International Prostatic Symptom Scoring (I-PSS).

I-PSS is one of the criteria to evaluate the severity of lower urinary tract symptoms described above, and is used to judge the severity by scoring responses to the following questions:
(1) During the last month, have you had a sensation of residual urine after urinating?
(2) During the last month, have you had to urinate again in less than two hours after you have urinated?
(3) During the last month, have you experienced interrupted urination?
(4) During the last month, have you found it difficult to postpone urination?
(5) During the last month, have you had a weak urinary stream?
(6) During the last month, have you had to strain to urinate?
(7) During the last month, how many times did you typically have to get up to urinate after you went to bed till you got up in the morning?
Out of these questions, the responses to (1) through (6) are scored as 0 if the response was not at all, 1 if less than 1 time in 5, 2 if less than 1 time in 2, 3 if about 1 time in 2, 4 if more than 1 time in 2, and 5 if almost always, and the responses to (7) are scored as 0, 1, 2, 3, 4, and 5 if the response was none, 1 time, 2 times, 3 times, 4 times, and 5 times or more, respectively.
The indices (1), (3), (5), and (6) are those to evaluate voiding symptoms, and (2), (4), and (7) are those to evaluate storage symptoms.
Patients whose total I-PSS scores are 8 or higher are diagnosed as severe enough to be treated.

As representative therapeutic drugs currently in use for prostatic hypertrophy, adrenaline α₁ receptor antagonists aimed to ameliorate the functional occlusion are widely known and being used clinically not only in Japan but also in the world over as drugs of the first choice for treatment of dysuria associated with prostatic hypertrophy. The mechanism of action of the adrenaline α₁ receptor antagonists is based on the notion that the receptor involved in the contraction of the prostate and urethral smooth muscle is the adrenaline α₁ receptor.
On the other hand, the prostate is a target organ of male hormones, androgens, which are intimately involved in the genesis and development of prostatic hypertrophy. As drugs to inhibit such androgens action, chlormadinone acetate and allylestrenol are used in Japan. In Europe and U.S., finasteride and dutasteride, which are drugs that inhibit the enzyme (5α reductase) that converts testosterone to dihydrotestosterone in the prostate, are used for treatment in order to improve dysuria associated with prostatic diminution.

So far, several combination therapies utilizing an adrenaline α receptor antagonist and a muscarinic receptor antagonist have been known to improve lower urinary tract symptoms associated with prostatic hypertrophy, but none of the combinations of an adrenaline α receptor antagonist and a muscarinic receptor antagonist have been known to be equal or superior to therapies with an adrenaline α receptor alone in efficacy to improve voiding symptoms in terms of I-PSS.

For example, there is a report in the literature which described efficacy of a therapy using a combination of doxazosin, an adrenaline α receptor antagonist, and propiverine hydrochloride, a muscarinic receptor antagonist, on lower urinary tract symptoms associated with prostatic hypertrophy (Non-patent document 1).
As shown in Table 3 of this document, doxazosin alone improved the total I-PSS by 7.3 points while the combination of doxazosin and propiverine hydrochloride improved it by 7.4 points. With respect to the storage symptom score, doxazosin alone improved it by 2.9 points whereas the combination of doxazosin and propiveine hydrochlorice improved it by 3.7 points. Namely, with respect to the total I-PSS and storage symptom scores, improvement by the combination therapy with the muscarinic receptor antagonist was better than or similar to improvement by the therapy with the adrenaline α receptor antagonist alone.
Regarding the voiding symptom score on the other hand, doxazosin alone improved it by 4.5 points whereas the combination of doxazosin and propiverine hydrochloride improved it by only 3.7 points, indicating that the combination with the muscarinic receptor antagonist lowered the improving effect compared with the adrenaline α receptor antagonist alone.

In view of its mechanism of action, it is expected that a muscarinic receptor antagonist may be effective in improving storage symptoms, but it could antagonize the improving effect on voiding symptoms, since it may inhibit contraction of the bladder at the time of urination. Actually, when improvement of lower urinary tract symptoms associated with prostatic hypertrophy is desired, a muscarinic receptor antagonist should be administered with great caution or it is contra-indicated because it may bring about urinary stasis or urinary retention that may necessitate catheterization or surgical operation (Non-patent document 2). For example, in Japan, a package insert of solifenacin succinate, a muscarinic receptor antagonist, states that it is contra-indicated "in patients with urinary retention" because "it may inhibit contraction of the bladder at the time of urination and thus deteriorate the symptom", and that "in patients with diseases accompanied with lower urinary tract occlusion (prostatic hypertrophy, and the like.), "it should be administered with great caution because "it may cause urinary retention by its anticholinergic action". The package insert also gives an important basic caution that "In patients with dysuria (lower urinary tract occlusion diseases [prostatic hypertrophy, and the like.] or urination muscle contraction disturbances, and the like.), residual urine volume should be measured before administration of this drug and special tests should be considered when necessary. Also, the patients should be kept under careful observation throughout the administration period by periodically confirming absence of aggravation of dysuria."
Therefore, in view of the possibility that a muscarinic receptor antagonist may worsen voiding symptoms owing to its own mechanism of action, one could expect the result reported in Non-patent document 1 that, while the combination therapy with the adrenaline α receptor antagonist and the muscarinic receptor antagonist further improved storage symptoms compared to the therapy with the adrenaline α receptor alone, the combination therapy antagonized the improving effect in voiding symptoms, namely, it exerted a negative effect on the voiding symptoms.
However, in the trial described in this document, the adrenaline α receptor antagonist and the muscarinic receptor antagonist were directly administered to patients with prostatic hypertrophy accompanied with overactive bladder symptoms (urgency, pollakiuria, urinary incontinence and/or nocturia, and the like.) without preliminary selection of test patients by administering an adrenaline α, receptor antagonist, and the like.

Also, as regard to combination therapies with an adrenaline α receptor antagonist and a muscarinic receptor antagonist for improvement of lower urinary tract symptoms associated with prostatic hypertrophy, there is a report on a combination therapy using tamsulosin as an α receptor antagonist and tolterodine as a muscarinic receptor antagonist (Non-patent document 3).
This document demonstrates that the combination therapy with tamsulosin and tolterodine significantly improved the total I-PSS compared to placebo, and that it also significantly improved, urgency, urination frequency in 24 hours, and nocturnal urination frequency each of which are the storage symptom indices, compared to placebo. However, there is no description about its improving efficacy on voiding symptoms.

Tamsulosin or its salt is known as an adrenaline α receptor antagonist (Patent document 1) and its chemical structure is shown below. Tamsulosin is also known as YM617.

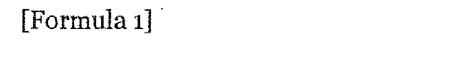

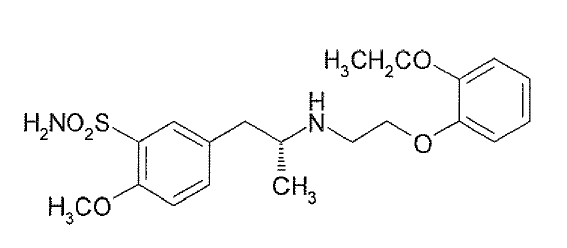

Solifenacin or its salt is known as a muscarinic receptor antagonist (Patent document 2) and its chemical structure is shown below. Solifenacin is also known as YM905.

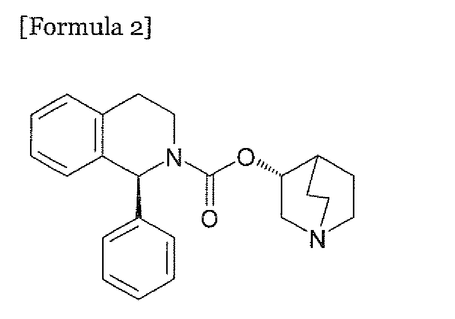

Patent document 1: US Patent No. 4,703,063
Patent document 2: International Publication WO No. 95/20194
Non-patent document 1: The Journal of Urology Vol. 174, p. 1334 (2005)
Non-patent document 2: Folia Pharmacologica Japonica Vol. 12, p. 331(2003)
Non-patent document 3: The Journal of the American Medical Association Vol. 296, No.19, p.2319 (2006)

### Disclosure of the Invention

### Problems to be Solved by the Invention

Pharmaceutical compositions containing tamsulosin or its pharmaceutically acceptable salts and solifenacin or its pharmaceutically acceptable salts, particularly agents for improvement of lower urinary symptoms associated with prostatic hypertrophy are provided.
In addition, combination use and combination therapy with tamsulosin, or its pharmaceutically acceptable salts, and solifenacin, or its pharmaceutically acceptable salts, for improvement of lower urinary tract symptoms associated with prostatic hypertrophy are provided.

### Means for Solving the Problems

The present inventors intensively studied improvement of lower urinary tract symptoms associated with prostatic hypertrophy, and as a result, discovered that the use of particularly tamsulosin, or its pharmaceutically acceptable salts, and solifenacin, or its pharmaceutically acceptable salts, exhibited unique improving effect on lower urinary tract symptoms associated with prostatic hypertrophy, and thus accomplished the present invention.

That is, the present invention relates to:

### [1]

A pharmaceutical composition containing, as active ingredients, (R)-5-(2-{[2-(2-ethoxyphenoxy)ethyl]amino}propyl-2-methoxybenzene-1-sulfonamide or its pharmaceutically acceptable salt and (1S)-1-phenyl-1,2,3,4-tetrahydroisoquinoline-2-carboxylic acid (3R)-quinuclidin-3-yl ester or its pharmaceutically acceptable salt;

### [2]

A pharmaceutical composition of [1] which is the pharmaceutical composition for improvement of lower urinary tract symptoms associated with prostatic hypertrophy;

### [3]

A pharmaceutical composition of [1] or [2] in which (R)-5-(2-{[2-(2-ethoxyphenoxy)ethyl]amino}propyl-2-methoxybenzene-1-sulfonamide or its pharmaceutically acceptable salt is (R)-5-(2-{[2-(2-ethoxyphenoxy)ethyl]amino}propyl-2-methoxybenzene-1-sulfonamide hydrochloride;

### [4]

A pharmaceutical composition of [1] to [3] in which (1S)-1-phenyl-1,2,3,4-tetrahydroisoquinoline-2-carboxylic acid (3R)-quinuclidin-3-yl ester or its pharmaceutically acceptable salt is (1S)-1-phenyl-1,2,3,4-tetrahydroisoquinoline-2-carboxylic acid (3R)-quinuclidin-3-yl ester succinate;

### [5]

A pharmaceutical composition for improvement of lower urinary tract symptoms associated with prostatic hypertrophy containing, as active ingredients, (R)-5-(2-{[2-(2-ethoxyphenoxy)ethyl]amino}propyl-2-methoxybenzene-1-sulfonamide hydrochloride and (1S)-1-phenyl-1,2,3,4-tetrahydroisoquinoline-2-carboxylic acid (3R)-quinuclidin-3-yl ester succinate;

### [6]

A pharmaceutical composition of [5] for improvement of lower urinary tract symptoms in patients with prostatic hypertrophy showing a total 1-PSS score of 8 or higher after administration of an adrenaline α receptor antagonist for 4 weeks or longer;

### [7]

A pharmaceutical composition of [5] or [6] in which (R)-5-(2-{[2-(2-ethoxyphenoxy)ethyl]amino}propyl-2-methoxybenzene-1-sulfonamide hydrochloride is 0.2 mg or 0.4 mg of [R)-5-[2-{[2-(2-ethoxyphenoxy)ethyl] amino}propyl-2-methoxybenzene-1sulfonamide hydrochloride;

### [8]

A pharmaceutical composition of [5] to [7] in which (1S)-1-pbenyl-1,2,3,4-tetrahydroisoquinoline-2-carboxylic acid (3R)-quinuelidin-3-yl ester succinate is 2.5 mg or 5.0 mg of (1S)-1-phenyl-1,2,3,4-tetrahydroisoquinoline-2-carboxylic acid (3R)-quinuclidin-3-yl ester succinate;

### [9]

A pharmaceutical composition of [5] to [7] in which (1S)-1-phenyl-1,2,3,4-tetrahydroisoquinoline-2-carboxylic acid (3R)-quinuclidin-3-yl ester succinate is 3 mg, 6 mg, or 9 mg of (1S)-1-phenyl-1,2,3,4-tetrahydroisoquinoline-2-carboxylic acid (3R)-quinuclidin-3-yl ester succinate;

### [10]

A method to improve lower urinary tract symptoms associated with prostatic hypertrophy consisting of administering (R)-5-(2-{[2-(2-ethoxyphenoxy)ethyl]amino}propyl-2-methoxybenzene-1-sulfonamide hydrochloride and (1S)-1-phenyl-1,2,3,4-tetrahydroisoquinoline-2-carboxylic acid (3R)-quinuclidin-3-yl ester succinate simultaneously or with a time interval.

Also, the present invention relates to a pharmaceutical composition for improvement of lower urinary tract symptoms associated with prostatic hypertrophy, which contains tamsulosin, or its pharmaceutically acceptable salt, and solifenacin, or its pharmaceutically acceptable salt, namely, an improving agent for lower urinary tract symptoms associated with prostatic hypertrophy, which contain tamsulosin, or its pharmaceutically acceptable salt, and solifenacin, or its pharmaceutically acceptable salt.
Also, the present invention relates to use of tamsulosin, or its pharmaceutically acceptable salt, and solifenacin, or its pharmaceutically acceptable salt, for manufacturing of a pharmaceutical composition for improvement of lower urinary tract symptoms associated with prostatic hypertrophy, and a method for improving lower urinary tract symptoms associated with prostatic hypertrophy comprising administering an effective dose of tamsulosin, or its pharmaceutically acceptable salt, and solifenacin, or its pharmaceutically acceptable salt to patients.

### Effect of the Invention

A pharmaceutical composition containing tamsulosin, or its pharmaceutically acceptable salt, and solifenacin, or its pharmaceutically acceptable salt, as active ingredients can be used as an agent to improve lower urinary tract symptoms associated with prostatic hypertrophy.

### Best Mode for Carrying Out the Invention

In the present description, "improvement of lower urinary tract symptoms associated with prostatic hypertrophy" means improvement of lower urinary tract symptoms in patients with benign prostatic hypertrophy, including (1) voiding symptoms such as weak stream, intermittency, delayed urination, and abdominal pressure urination, (2) storage symptoms such as pollakiuria, nocturia, and urgency, and (3) post-voiding symptoms such as emptying and post-urination dribbling.
Also, as the "adrenaline α receptor antagonists", tamsulosin, doxazosin, alfuzosin, bunazosin, indolamine, naphthopidil, prazosin, terazosin, urapidil, and silodosin can be exemplified.

Tamsulosin or its pharmaceutically acceptable salt, an effective ingredient of a pharmaceutical composition of the present invention, is easily available by methods described, for example, in the aforementioned patent document 1, or by methods obvious to the person skilled in the art, or by their modifications.
Solifenacin or its pharmaceutically acceptable salt, an effective ingredient of a pharmaceutical composition of the present invention, is easily available by methods described, for example, in the aforementioned patent document 2, or by methods obvious to the person skilled in the art, or by their modifications.

The "pharmaceutically acceptable salts" in "tamsulosin or its pharmaceutically acceptable salt" and "solifenacin or its pharmaceutically acceptable salt" mean those salts described in the aforementioned Patent document 1 or the aforementioned Patent document 2, respectively. Their specific examples are addition salts with inorganic acids such as hydrochloric acid, hydrobromic acid, hydroiodic acid, sulfuric acid, nitric acid, and phosphoric acid, or with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid, and salts with inorganic bases such as sodium, potassium, magnesium, calcium, aluminium, or with organic bases such as methylamine, ethylamine, ethanolamine, lysine, and ornithine, and salts with various amino acids and amino acid derivatives such as acetylleucine, ammonium salts, and others.

Furthermore, the "pharmaceutically acceptable salts" in "tamsulosin or its pharmaceutically acceptable salt" and "solifenacin, or its pharmaceutically acceptable salt" may be various hydrates, solvates and polymorphic crystalloids, which are all included as an active ingredient of the pharmaceutical composition of the present invention. The present invention also includes a pharmaceutical composition containing various radioactive or non-radioactive isotope-labeled compounds.

Embodiments of the present invention are described below.

(1) Among pharmaceutical compositions or methods to improve lower urinary tract symptoms associated with prostatic hypertrophy in the present invention, a pharmaceutical composition to improve lower urinary tract symptoms or a method for improving lower urinary tract symptoms associated with prostatic hypertrophy in patients with prostatic hypertrophy whose total I-PSS scores are 8 or higher after administration of an adrenaline α receptor antagonist for 4 weeks or longer.
(2) Among pharmaceutical compositions or methods to improve lower urinary tract symptoms associated with prostatic hypertrophy in the present invention, a pharmaceutical composition in which tamsulosin or its pharmaceutically acceptable salt is tamsulosin hydrochloride ((R)-5-(2-{[2-(2-ethoxyphenoxy)ethyl]amino}propyl-2-methoxybenzene-1-sulfonamide hydrochloride) or 0.1 mg to 0.8 mg tamsulosin hydrochloride, or 0.1 mg, 0.2 mg, 0.4 mg, or 0.8 mg tamsulosin hydrochloride, or a method to improve lower urinary tract symptoms associated with prostatic hypertrophy.
(3) Among pharmaceutical compositions or methods to improve lower urinary tract symptoms associated with prostatic hypertrophy in the present invention, a pharmaceutical composition in which solifenacin or its pharmaceutically acceptable salt is solifenacin succinate ((1S)-1-phenyl-1,2,3,4-tetrahydroisoquinoline-2-carboxylic acid (3R)-quinuclidin-3-yl ester succinate), or 2.5 mg to 10 mg solifenacin succinate, or 2.5 mg, 3 mg, 5 mg, 6 mg, 9 mg, or 10 mg solifenacin succinate in order to improve lower urinary tract symptoms in patients with prostatic hypertrophy, or a method to improve lower urinary tract symptoms associated with prostatic hypertrophy.
(4) Among pharmaceutical compositions of the present invention, a pharmaceutical composition which is orally administered.
(5) Among methods to improve lower urinary tract symptoms associated with prostatic hypertrophy according to the present invention, a method to improve lower urinary tract symptoms associated with prostatic hypertrophy by oral drug administration.
(6) Among above (1) through (5), a combination of 2 or more pharmaceutical compositions or a method to improve lower urinary tract symptoms associated with prostatic hypertrophy.

The pharmaceutical composition of the present invention can be prepared by usually employed methods with tamsulosin or its pharmaceutically acceptable salt and solifenacin or its pharmaceutically acceptable salt along with carriers, excipients, or other additives for usual drug formulation. Administration can be performed orally in the forms of tablets, pills, capsules, granules, powders, liquids and the like, or parenterally in the forms of intra-articular, intravenous, or intramuscular injections, suppositories, ophthalmic solutions, ointments, percutaneous liquids, ointments, transdermal patches, transmucosal liquids, transmucosal patches, or inhalants. Oral administration can be cited as a preferred aspect.

As solid compositions for oral administration, tablets, powders, granules, and the like are used. In these solid compositions, the effective ingredient is mixed with at least one of inert excipients, and the like. The composition may follow conventional methods to contain inert additives such as lubricants, disintegrators, stabilizers, or solubilizers. If necessary, tablets and pills may be covered with sugar or gastric- or enteric-coating films.
Liquid compositions for oral administration contain pharmaceutically acceptable emulsifiers, solubilizers, suspensions, syrups, or elixirs, and the like. They also contain generally employed inert diluents such as purified water or ethanol. These liquid compositions may also, in addition to inert diluents, adjuvants such as solubilizers, humidifiers or suspensions, or sweetening agents, flavoring agents, aromatic agents, or antiseptic agents.

Injections for parenteral administration contain aseptic aqueous or non-aqueous solubilizers, suspensions, or emulsifiers. Aqueous solvents include, for example, distilled water for injection or physiological saline. As non-aqueous solvents, there are propylene glycol, polyethylene glycol, vegetable oils such as olive oil, alcohols such as ethanol, and Polysorbate 80 (Japanese Pharmacopea), and the like. Such compositions may further contain isotonizing agents, antiseptic agents, humidifying agents, emulsifying agents, dispersing agents, stabilizing agents or solubilizing agents. These may be sterilized by filtration through, for example, a bacterium-retaining fitter, addition of a sterilizer, or irradiation. These may also be prepared by preparing solid compositions aseptically and then solubilizing or suspending them in sterilized water or a sterilized solvent for injection.

Topical preparations include ointments, plasters, creams, jellies, epithems, nebulas, lotions, ophthalmic solutions or ointments, and the like. They contain generally employed ointment bases, lotion bases, aqueous or non-aqueous liquids, suspending agents, emulsifying agents, and the like. The ointment and lotion bases include, for example, polyethylene glycol, propylene glycol, white petrolatum, bleached beeswax, polyoxyethylene- hardened caster oil, glycerin monostearate, stearyl alcohol, cetyl alcohol, lauromacrogol, sorbitan sesquioleate, and the like.

Transmucosal agents such as inhalants or transnasal agents utilize solids, liquids, or semi-liquids, and they can be prepared by conventional methods. For example, known excipients, or further, pH adjusters, antiseptics, surfactants, lubricants, stabilizers, or thickners may properly be added. Drug administration may be aided by appropriate devices for inhalation or insufflation. For example, by using known devices such as pre-measured inhalation devices or nebulizers, a compound can be administered alone or as a formulated mixture in a powder, or in combination with pharmaceutically acceptable carriers in a solution or suspension. Dry-powder inhalers may be for single or multiple dosage of dry powder or powder-containing capsules, or may be assisted by a pressurized aerosol sprayer utilizing appropriate gases such as chlorofluoroalkanes, hydrofluoroalkanes, or carbon dioxide as propelling agents.

Combination administration in the present invention may be performed by administering two drugs simultaneously, one immediately after the other, or with a desired time interval between them. When administered simultaneously, a combination drug that is a single drug in which both effective ingredients are contained, or two separate formulations each of which contains each effective ingredient may be administered.

### Examples

Efficacy of the combination therapy with the effective ingredients of the pharmaceutical compositions according to the present invention has been confirmed by the following studies.

### (Study 1)

Patients with prostatic hypertrophy who still had symptoms of overactive bladder (urgency, pollakiuria, urinary incontinence and/or nocturia, and the like.) after treatment with an α receptor antagonst for 4 weeks or longer were allocated for administration to two treatment groups: (1) tamsulosin hydrochloride 0.2 mg (hereinafter referred to as "Single Group"), and (2) tamsulosin hydrochloride 0.2 mg and solifenacin succinate 2.5 mg (hereinafter referred to as "Combination Group"), and then their symptoms were evaluated by I-PSS after 4 weeks of treatment.
The results are shown below.

**[Table 1]**

| | | Before treatment | After treatment of 4 weeks | Change |
|---|---|---|---|---|
| Total scores of I-PSS | Single | 11.6 | 10.8 | -0.8 |
| | Combination | 13.9 | 11.2 | -2.7 |
| Scores of storage symptoms (1) (3) (5) (6) | Single | 6.5 | 5.8 | -0.7 |
| | Combination | 7.2 | 5.5 | -1.7 |
| Scores of voiding symptoms (2) (4) (7) | Single | 4.2 | 4.2 | 0.0 |
| | Combination | 5.3 | 4.6 | -0.6 |

Since the score of I-PSS becomes greater as the symptom worsens, greater minus change in scores indicates greater efficacy for the symptoms.
As shown in Table, the combination therapy with tamsulosin hydrochloride and solifenacin succinate showed improvement effect on the total I-PSS and storage symptom scores in comparison with the single therapy with tamsulosin hydrochloride, and it gave no effect on the voiding symptom score unexpectedly, or it even gave a further improving tendency in comparison with the single therapy with tamsulosin hydrochloride.
Namely, it has become clear that the combination therapy with tamsulosin hydrochloride and solifenacin succinate did not worsen the voiding symptom index in comparison with the single therapy with tamsulosin hydrochloride.

### (Study 2)

In new patients and those with prostatic hypertrophy and remaining overactive bladder symptoms after treatment with tamsulosin hydrochloride for 4 weeks or longer, the following treatments were performed.
(1) New patients were treated with 0.2 mg tamsulosin hydrochloride for 4 weeks.
(2) Out of the new patients who were treated with 0.2 mg tamsulosin hydrochloride for 4 weeks, those who still needed treatment for their overactive bladder symptoms, and the patients who received tamsulosin hydrochloride for 4 weeks or longer but still showed overactive bladder symptoms were treated with 0.2 mg tamsulosin hydrochloride and 2.5 mg solifenacin succinate for 8 weeks.
(3) For those who received solifenacin succinate for 4 weeks without enough efficacies, the dose of solifenacin succinate was raised to 5.0 mg for the remaining 4 weeks according to the patients' intention.
Their symptoms were evaluated by 1-PUSS before and after the treatment with solifenacin succinate for 8 weeks. The results are shown in Table 2.

**[Table 2]**

| | Before combination therapy | After 8 weeks of combination therapy | Change |
|---|---|---|---|
| Total scores of I-PSS | 14.5 | 10.6 | -3.9 |
| Scores of storage symptoms (1) (3) (5) (6) | 7.9 | 4.8 | -3.1 |
| Scores of voiding symptoms (2) (4) (7) | 5.1 | 5.0 | -0.1 |

As shown in Table 2, the combination therapy with tamsulosin hydrochloride and solifenacin succinate showed improving efficacy on the total I-PSS and storage symptom scores in comparison with the single therapy with tamsulosin hydrochloride, and furthermore it gave no effect on the voiding symptom score unexpectedly in comparison with the single therapy with tamsulosin hydrochloride.
Namely, it has become clear that the combination therapy with tamsulosin hydrochloride and solifenacin succinate did not worsen the voiding symptom index in comparison with the single therapy with tamsulosin hydrochloride.

### (Study 3)

Similar evaluation can be performed similarly except that the dose of tamsulosin hydrochloride is changed to 0.2 mg or 0.4 mg, and the dose of solifenacin succinate is changed to 2.5 mg, 5 mg, or 10 mg, or 3 mg, 6 mg, or 9 mg in Study 1 and Study 2.

Contrary to the notion that while the combination therapy with an adrenaline α receptor antagonist and a muscarinic receptor antagonist further improves storage symptoms in comparison with the single therapy with an adrenaline α receptor antagonist, it inhibits the improvement, namely, exerts a negative effect on voiding symptoms, the above results have unexpectedly demonstrated that the unique combination of tamsulosin or its pharmaceutically acceptable salt with solifenacin or its pharmaceutically acceptable salt neither interfere with nor influence the improving effect of tamsulosin on voiding symptoms in comparison with the improving effect of the single therapy with tamsulosin or its pharmaceutically acceptable salt alone on voiding symptoms, and that it exerts improving effect on the total I-PSS and storage symptom scores.

### Industrial Applicability

A pharmaceutical composition containing tamsulosin or its pharmaceutically acceptable salt and solifenacin or its pharmaceutically acceptable salt as active ingredients can be used as a drug for improvement of lower urinary tract symptoms associated with prostatic hypertrophy.

## Claims

1. A pharmaceutical composition, comprising (R)-5-(2-{[2-(2-ethoxyphenoxy)ethyl]amino}propyl-2-methoxybenzene-1-sulfonamide or its pharmaceutically acceptable salt, and (1S)-1-phenyl-1,2,3,4-tetrahydroisoquinoline-2-carboxylic acid (3R)-quinuclidin-3-yl ester or its pharmaceutically acceptable salt as active ingredients.

2. A pharmaceutical composition according to claim 1, which improves lower urinary tract symptoms associated with prostatic hypertrophy.

3. A pharmaceutical composition according to claim 1 or claim 2, wherein (R)-5-(2-{[2-(2-ethoxyphenoxy)ethyl]amino}propyl-2-methoxybenzene-1-sulfonamide or its pharmaceutically acceptable salt is (R)-5-(2-{[2-(2-ethoxyphenoxy)ethyl]amino}propyl-2-methoxybenzene-1-sulfonamide hydrochloride.

4. A pharmaceutical composition according to claim 1 to claim 3, wherein (1S)-1-phenyl-1,2,3,4-tetrahydroisoquinoline-2-carboxylic acid (3R)-quinuclidin-3-yl ester or its pharmaceutically acceptable salt is (1S)-1-phenyl-1,2,3,4-tetrahydroisoquinoline-2-carboxylic acid (3R)-quinuelidin-3-yl ester succinate.

5. A pharmaceutical composition for improving lower urinary tract symptoms associated with prostatic hypertrophy, comprising (R)-5₋(2-{[2-(2-ethoxyphenoxy)ethyl]amino}propyl-2-methoxybenzene-1-sulfonamide hydrochloride and (1S)-1-phenyl-1,2,3,4-tetrahydroisoquinoline-2-carboxylic acid (3R)-quinuclidin-3-yl ester succinate as active ingredients.

6. A pharmaceutical composition according to claim 5 for improving lower urinary tract symptoms in patients with prostatic hypertrophy whose total score of I-PSS is 8 or higher after administration of an adrenaline α receptor antagonist for 4 weeks or longer.

7. A pharmaceutical composition according to claim 5 or claim 6, wherein (R)-5-(2-{[2-(2-ethoxyphenoxy)ethyl]amino}propyl-2-methoxybenzene-1-sulfonamide hydrochloride is 0.2 mg or 0.4 mg of (R)-5-(2-{[2-(2-ethoxyphenoxy)ethyl]amino}propyl-2-methoxybenzene-1-sulfonamide hydrochloride.

8. A pharmaceutical composition according to claim 5 to claim 7, wherein (1S)-1-phenyl-1,2,3,4-tetrahydroisoquinoline-2-carboxylic acid (3R)-quinuclidin-3-yl ester succinate is 2.5 mg or 5.0 mg of (1S)-1-phenyl-1,2,3,4-tetrahydroisoquinoline-2-carboxylic acid (3R)-quinuclidin-3-yl ester succinate.

9. A pharmaceutical composition according to claim 5 to claim 7, wherein (1S)-1-phenyl-1,2,3,4-tetrahydroisoquinoline-2-carboxylic acid (3R)-quinuclidin-3-yl ester succinate is 3 mg, 6 mg, or 9 mg of (1S)-1-phenyl-1,2,3,4-tetrahydroisoquinoline-2-carboxylic acid (3R)-quinuclidin-3-yl ester succinate.

10. A method to improve lower urinary tract symptoms associated with prostatic hypertrophy comprising administering of (R)-5-(2-{[2-(2-ethoxyphenoxy)ethyl]amino}propyl-2-methoxybenzene-1-sulfonamide hydrochloride and (1S)-1-phenyl-1,2,3,4-tetrahydroisoquinoline-2-carboxylic acid (3R)-quinuclidin-3-yl ester succinate simultaneously or with a time interval.
